# EUROPEAN PATENT SPECIFICATION

(11) **EP 4 222 190 B1**
(45) Date of publication and mention of the grant of the patent: **09.12.2026**
(21) Application number: 21778517.9
(22) Date of filing: 30.09.2021
(51) Int. Cl.: C08G 73/02, C12M 1/12, A61K 9/20

(54) **AMORPHOUS THERMORESPONSIVE POLYMERS**
AMORPHE THERMOREAKTIVE POLYMERE
POLYMÈRES THERMOSENSIBLES AMORPHES

(30) Priority: 30.09.2020 EP 20199143
(43) Date of publication of application: 09.08.2023
(73) Proprietor: Universiteit Gent, 9000 Gent (BE)
(72) Inventor: RETAMERO DE LA ROSA, Victor, 9000 Gent (BE); HOOGENBOOM, Richard, 4533 AB Terneuzen (NL); WIERINGA, Paul, Andrew, 6217 JG Maastricht (NL); MORONI, Lorenzo, 6211 RL Maastricht (NL)
(74) Representative: Arnold & Siedsma
(86) International application number: PCT/EP2021/076951
(87) International publication number: WO 2022/069635

(56) References cited:
- WO-A1-2019/175434
- US-A1- 2013 323 188
- YOUNGEE SEO ET AL: "Poly(2-oxazoline) block copolymer based formulations of taxanes: effect of copolymer and drug structure, concentration, and environmental factors : POLY(2-OXAZOLINE) BASED FORMULATIONS OF TAXANES", POLYMERS FOR ADVANCED TECHNOLOGIES., vol. 26, no. 7, 1 July 2015 (2015-07-01), GB, pages 837 - 850, XP055496140, ISSN: 1042-7147, DOI: 10.1002/pat.3556
- NATALIA OLESZKO-TORBUS ET AL: "Control of the Crystalline Properties of 2-Isopropyl-2-oxazoline Copolymers in Condensed State and in Solution Depending on the Composition", MACROMOLECULES, vol. 50, no. 19, 18 September 2017 (2017-09-18), Washington DC United States, pages 7636 - 7645, XP055496379, ISSN: 0024-9297, DOI: 10.1021/acs.macromol.7b01639
- LÜBTOW M. ET AL: "Like dissolves like ? A comprehensive evaluation of partial solubilty parameters to predict polymer-drug compatibilty in ultrahigh drug-loaded polymer micelles", BIOMACROMOLECULES, vol. 20, 24 June 2019 (2019-06-24), pages 3041 - 3056, XP002802192, DOI: 10:1021/acs.biomac.9b00618

## Description

### FIELD OF THE INVENTION

The present invention relates to amorphous thermoresponsive polymers (ATP) and uses thereof in various domains, such as 3D printing and tissue engineering. Furthermore, the present invention relates to compositions, multidimensional structures and coatings comprising said polymer.

### BACKGROUND TO THE INVENTION

Thermo-responsive polymers belong to the class of stimuli-responsive materials and are well known for their sensitivity to temperature. Usually, slight environmental changes (e.g. slight temperature variations) are sufficient to induce significant changes relating to the polymer's properties. A number of application domains benefitting from these inducible property modifications are widely known, including the production of biodegradable packaging and drug delivery products (WO2019175434). Besides that, thermo-responsive polymers are extremely useful in the domain of biomedical research as biomaterials for bio fabrication such as tissue engineering and 3D cell culture applying 3D (bio)printing.

A 3D cell culture is an artificially created space for cells to grow and interact in three dimensions with the aim of producing physiologically relevant cellular structures closely resembling *in vivo* structures (e.g. tissue). In some cases, a structural support for cell attachment and tissue development may be required for 3D cell culture, more commonly referred to as scaffolds. Where a scaffold is intended to be degraded or removed from the cell structure, at a particular point in time, it is termed a sacrificial template. A number of biodegradable biological materials (e.g. gelatin, fibrinogen, collagen or alginate) may be used, in the production of such (sacrificial) template/scaffold. However, these have important drawbacks, such as poor stability and weak mechanical properties, preventing them from being used as standalone cell scaffold materials.

Other alternatives are fully synthetic materials (e.g. polystyrene, polyglycolic acid, polylactic acid and polycaprolactone) having improved mechanical properties and stability compared to the biodegradable biological materials. However, other problems arise when using these synthetic materials such as degradation rates that are difficult to control, bad tolerability by cells and the need of additional excipients to improve the stability and/or robustness of the materials.

None of these materials is able to provide a stable, robust scaffold for 3D cell culture which is well tolerated by cells whilst being removable upon demand by using a simple stimulus (e.g. temperature). There is thus a need in the art of such materials which can be applied for example in bio fabrication, more specifically serving as scaffolds for cellular growth.

This is where the specific properties of thermo-responsive polymers provide a solution to the aforementioned problems. At particular temperatures, these polymers are able to promote cell attachment and growth due to their hydrophobic properties. By contrast, these polymers become hydrophilic and removable after their specific transition temperatures are exceeded. This transition temperature is generally known as cloud point temperature (TCP). When the polymers become hydrophilic below their TCP, they are regarded as polymers exhibiting a lower critical solution temperature (LCST).

A number of thermo-responsive polymers have already been studied in the context of 2D cell culturing, wherein the polymers are deposited as a flat layer allowing for temperature-triggered switchable cell adhesion. However, the same materials were generally found unsuitable for more complex techniques such as melt electro writing (MEW) because of their limited mechanical properties and consequent inability to maintain the intended scaffold topology.

For example, the widely studied Poly(N-isopropylacrylamide) (PNIPAM) is known to exhibit a marked thermal hysteresis in the solubility phase transition (Halperin et al., 2015). PNIPAM redissolution upon cooling from a temperature above its TCP has been described in terms of "partial vitrification" (Van Durme et al., 2004).

Unless provided otherwise, the term "hysteresis" should be understood as the dependence of the state of a system on its history. It can often be linked with irreversible thermodynamic change such as phase transitions or internal friction. For PNIPAM this hysteresis results from the glassy hydrophobic state that is present above the transition temperature, limiting the redissolution upon cooling for larger macroscopic structures. Tailoring PNIPAM transition temperature is not straightforward, it is difficult to process as a melt due to the high glass transition temperature, and solvent electrospun fibers form ribbons instead of cylinders (Schoolaert et al., 2017).

Alternative thermoresponsive polymers such as, poly(oligoethylene glycol) acrylates (POEGAs and POEGMAs) have poor mechanical properties and cannot be used as free-standing scaffold materials.

Another thermoresponsive polymer widely employed in biomaterials, poly(N-vinylcaprolactam) (PNVCL or PVCL) swells in contact with water leading to a sharp decrease in its glass transition temperature (T_{g}) to below 0°C. Therefore, when hydrated, the polymer has poor mechanical properties precluding its applicability in 3D cell scaffolding.

Finally, the most widely used thermo-responsive polymers in biomedicine, Pluronics, have very weak mechanical properties and exhibit low long-term cell viability. To improve on these issues, researchers have added cross-linkable PEG and biologic materials such as hyaluronic acid but there is currently no commercial solution. Pluronics are deployable as a shearthinning gel-in-gel bioprinting medium for indirect solid freeform fabrication (SFF) (e.g. 3D

(bio)printing) but are generally not suited to be used as scaffolds for cell culture.

Polyoxazolines are a type of polymers widely studied due to their biocompatibility and tunable properties. The thermoresponsive properties of polyoxazoline derivatives are well-known in the art, and their use as materials for controlled cell-adhesion has been proposed. However, the application of polyoxazolines is to date limited to films on bidimensional surfaces due to the poor mechanical properties of these materials (Ryma et al., 2019). Recent attempts to overcome these issues by copolymerization have been unsuccessful. Indeed, as observed in other polymers discussed, when thermoresponsive polyoxazoline mats and molds were submerged in water at a temperature above the TCP, they remained undissolved but lost their shape stability (Oleszko-Torbus et al., 2020).

Polyoxazoline copolymers are also prepared in document US 2013/323188, wherein their use is described in compositions for the treatment of oxidative stress diseases. In particular, a process for the preparation of a random copolymer of poly(2-ethyl-2-oxazoline) and (2-butyl-2-oxazoline) is disclosed.

The current invention relates to a group of amorphous thermoresponsive polymers (ATP), namely polyoxazoline derivates with a variable copolymer composition. These are characterized by switchable hydrophobic/hydrophilic properties coinciding with the transition temperatures of the specific copolymer composition and are not associated with any significant loss of material shape nor swelling or hysteresis upon exposure to water above the TCP. Because of these properties, the amorphous thermoresponsive polymers tackle a number of disadvantages of prior art materials as mentioned above. By lowering the temperature, the polyoxazoline derivates of the present invention are able to switch from hydrophobic to hydrophilic and rapidly dissolve. The ability of rapidly switching between these hydrophobic and hydrophilic properties by varying temperature offers great advantages over existing polymers. Furthermore, these polyoxazoline derivates are able to promote cell adhesion and growth in the hydrophobic state without the need of adding any biological material (e.g. collagen).

These properties make them perfectly suitable for a number of application domains, such as the construction of tubular structures for cell culture and tissue engineering (using 3D (bio)printing), application in coatings and sustained release formulations.

### SUMMARY OF THE INVENTION

In a first aspect, the present invention relates to an amorphous thermoresponsive polymer (ATP); in particular a PsecBuOx-stat-PAOx co-polymer represented by formula (I); 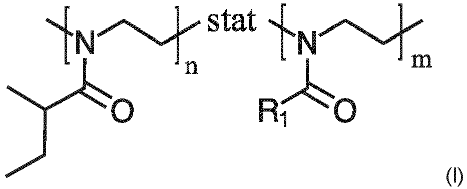 wherein each R₁ is independently selected from -methyl, -ethyl, n-propyl, cyclo-propyl and iso-propyl; and the sum of m and n is between about 20 - 1000, preferably between 200 - 700, most preferably between 300 - 500.

In a second embodiment, said co-polymer may be represented by formula (la) 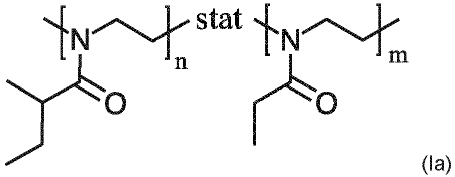 wherein the sum of m and n is between about 20 - 1000, preferably between 200-700, most preferably between 300 - 500.

In a following embodiment, said co-polymer may be represented by formula (Ib) 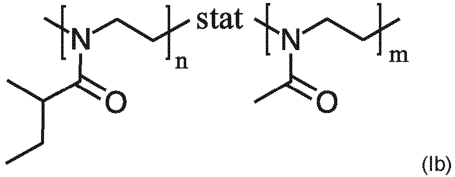 wherein the sum of m and n is between about 20 - 1000, preferably between 200-700, most preferably between 300 - 500.

In a next embodiment, said co-polymer may comprise a ratio of PsecBuOx monomeric units to PMeOx, PEtOx, PnPrOx, PcPrOx and/or PiPrOx monomeric units is about and between 5/95 mol% and 95/5 mol%.

In another embodiment, said co-polymer may comprise a ratio of PsecBuOx monomeric units to PMeOx, PEtOx, PnPrOx, PcPrOx and/or PiPrOx monomeric units is about and between 50/50 - 80/20 mol%.

In a following embodiment, said co-polymer may comprise a ratio of PsecBuOx monomeric units to PMeOx, PEtOx, PnPrOx, PcPrOx and/or PiPrOx monomeric units is about and between 20/80 - 50/50 mol%.

In a next aspect, a composition comprising said co-polymer is disclosed.

In a further embodiment, the present invention provides the compositions and/or copolymers as defined herein for use in human or veterinary medicine.

In yet another embodiment, the present invention provides the compositions and/or copolymers as defined herein for use in manufacturing of a 2D or 3D structure; more in particular in the manufacturing of a sacrificial template.

In a next embodiment, the present invention provides the compositions and/or copolymers as defined herein for use in a method selected from the list comprising: electrospinning (ES), melt electrospinning (MES), melt electrowriting, additive manufacturing, fused deposition modelling, thermoforming, casting and 3D printing.

In another embodiment, the present invention provides the compositions and/or copolymers as defined herein for use in tissue engineering, implant manufacturing, *in vitro* cell cultures, and/or the manufacturing of an in vitro cell-culture scaffold.

In yet another embodiment, the present invention provides the compositions and/or copolymers as defined herein for use as a coating.

In a further embodiment, the present invention provides the compositions and/or copolymers as defined herein for use in as a drug formulation is disclosed.

A next aspect relates to a 2D or 3D structure comprising said co-polymer.

A further aspect relates to a coating comprising said co-polymer.

### BRIEF DESCRIPTION OF THE DRAWINS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings making apparent to those skilled in the art how the several forms of the invention may be embodied in practice.

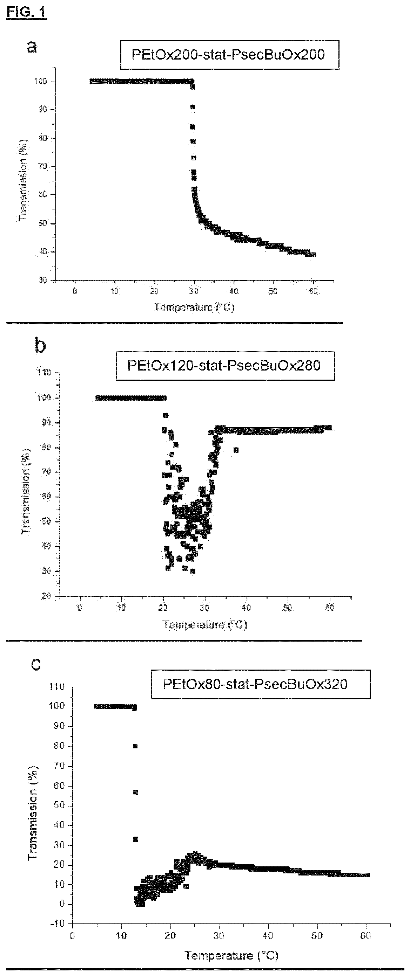

**FIG. 1** discloses turbidimetry measurements -2^{nd} heating ramp- of PEtOx200-stat-PsecBuOx200 (a) showing a T_{CP} of ca. 30 °C, PEtOx120-stat-PsecBuOx280 (b) showing a T_{CP} of ca. 20 °C, and PEtOx80-stat-PsecBuOx320 (c) showing a T_{CP} of ca. 13 °C according to an embodiment of the current invention.

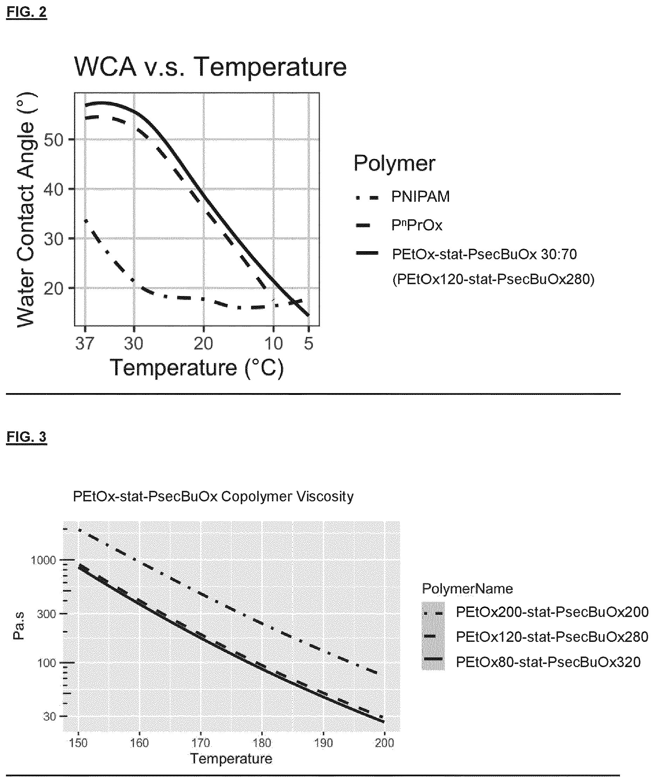

**FIG. 2** discloses water contact angle (WCA) measurements of PNIPAM, PnPrOx and PEtOx120-stat-PsecBuOx280. This was measured by spin coating a thin film of the polymer (dissolved in chloroform) onto a glass coverslip and then placing this coating coverslip onto a flat Peltier element. This was incorporated into a WCA measurement system along with a heated syringe of PBS which maintained PBS solution at approximately 60°C. A drop of this warmed PBS was deposited onto the polymer film and the WCA was monitored via time lapse over a 2 minute period until the angle had stabilize. The angle was automatically determined according to the proprietary software of the WCA system. The WCA of the last 30 seconds was observed to be stable and, therefore, averaged to produce the 'final' WCA. This was measured for a number of temperatures ranging from 37°C to 5°C, reflecting the transition from cell incubator to refrigerator, respectively.
**FIG. 3** discloses melt viscosity measurements performed for the different PEtOx-stat-PsecBuOx variants. The measurements were performed in a parallel plate rheometer with heated plates. 100 mg of each polymer were loaded between the plates, the temperature was raised to 200°C to initially melt the polymer, and then the viscosity was determined by measuring the rotational force required to subject the polymer melt to a cyclic of 1° angular displacement. The sample was then cooled slowly by 1°C/min.

### DETAILED DESCRIPTION OF THE INVENTION

As already detailed herein above, in a first aspect, the present invention relates to hydrophobic thermoresponsive polymers, in particular comprising a PsecBuOx-PAOx backbone; wherein PAOx represents poly(2-alkyl-2-oxazoline), and alkyl may be selected from any one of methyl, ethyl, -n-propyl, cyclo-propyl and iso-propyl, or combinations thereof.

More in particular the present invention provides a PsecBuOx-stat-PAOx co-polymer represented by formula (I); 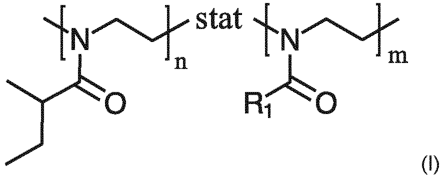 wherein R₁ is selected from -methyl, -ethyl, -n-propyl, cyclo-propyl and iso-propyl; and the sum of m and n is between about 20 - 1000, preferably between 200 - 700, most preferably between 300 - 500.

Alternatively, in formula (I), R₁ is C₁₋₃alkyl; and the sum of m and n is between about 20 - 1000, preferably between 200 - 700, most preferably between 300 - 500. The term "alkyl" by itself or as part of another substituent refers to a fully saturated hydrocarbon of Formula CₓH₂ₓ₊₁ wherein x is a number greater than or equal to 1. Generally, alkyl groups of this invention comprise from 1 to 3 carbon atoms. Alkyl groups may be linear, cyclic or branched and may be substituted as indicated herein. When a subscript is used herein following a carbon atom, the subscript refers to the number of carbon atoms that the named group may contain. Thus, for example, C₁₋₃alkyl means an alkyl of one to three carbon atoms. Examples of alkyl groups are methyl, ethyl, n-propyl, i-propyl. c-propyl.

Unless provided otherwise, the term "stat" should be understood as "statistical", referring to statistical polymers, in this case being formed by one-pot statistical co-polymerization, e.g. of said monomer units as disclosed in formula (I). Co-polymerization finds its particular use in the current invention in tuning the transition temperature of said co-polymers as variation of the ratio of both comonomers leads to a change in transition temperature. Incorporation of more secBuOx leads to a decrease in transition temperature.

Poly(2-oxazoline)s or poly(2-alkyl/aryl-2-oxazolines), are commonly abbreviated as PAOx, PAOs, POx or POZ and are readily obtained *via* the cationic ring- opening polymerization of 2-oxazolines. This type of polymers is widely studied due to their biocompatibility and tunable properties. The thermoresponsive properties of polyoxazoline derivatives are well-known in the art and are directly dependent on the specific substitution of the polymer. The aqueous solubility of this polymer type ranges from the highly hydrophilic poly(2-methyl-2-oxazoline) (PMeOx) which does not exhibit an LCST (lower critical solution temperature) behavior to the water insoluble poly(2-n-butyl-2-oxazoline) (Table 1).The specific LCST value of the polymer can be systematically modified by copolymerization of two differently substituted cyclic iminoether monomers.

They are for instance described in WO2013103297 and WO2019175434.
Poly(2-oxazoline) polymers can be described by the general formula Z 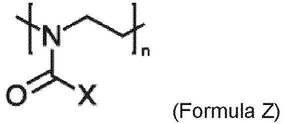 wherein X is a linear or branched C₁₋₅alkyl or a cyclopropyl.

Depending on the nature of X, the polymer will show a different hydrophobicity and LCST.

Representative polyoxazolines are shown in Table 1.

**Table 1: Representative polyoxazolines**

| **X** | **Abbreviation** | **LCST** |
|---|---|---|
| Methyl | PMeOx | No LCST |
| Ethyl | PEtOx | ~60 °C |
| iso-Propyl | PiPrOx | ~35 °C |
| cyclo-Propyl | PcPrOx | ~30 °C |
| n-Propyl | PnPrOx | ~25 °C |
| sec-Butyl | PsecBuOx | ~ 0-5 °C |
| n-Butyl | PnButOx | Water insoluble |

The lower critical solution temperature (LCST) is the transition temperature where the polymer reversibly transitions from hydrophilic to hydrophobic upon heating.

In the current invention, more specifically, PMeOx corresponds to poly(2-methyl-2-oxazoline), PEtOx corresponds to poly(2-ethyl-2-oxazoline), PnPrOx corresponds to poly(2-n-propyl-2-oxazoline), PiPrOx corresponds to poly(2-iso-propyl-2-oxazoline), PcPrOx corresponds to poly(2-c-propyl-2-oxazoline) and PsecBuOx corresponds to poly(2-sec-butyl-2-oxazoline).

In a specific embodiment, the PAOx of the present invention may also be a combination of PMeOx and PEtOx, thereby rendering a PsecBuOx/PMeOx/PEtOx co-polymer, a combination of PMeOx and PnPrOx, thereby rendering a PsecBuOx/PMeOx/PnPrOx co-polymer, a combination of PMeOx and PiPrOx, thereby rendering a PsecBuOx/PMeOx/PiPrOx copolymer, a combination of PMeOx and PcPrOx, thereby rendering a PsecBuOx/PMeOx/PcPrOx co-polymer, a combination of PEtOx and PnPrOx, thereby rendering a PsecBuOx/PEtOx/PnPrOx co-polymer, a combination of PEtOx and PiPrOx, thereby rendering a PsecBuOx/PEtOx/PiPrOx co-polymer, a combination of PEtOx and PcPrOx, thereby rendering a PsecBuOx/PEtOx/PcPrOx co-polymer, a combination of PMeOx, PEtOx and PnPrOx, thereby rendering a PsecBuOx/PMeOx/PEtOx/PnPrOx co-polymer, a combination of PMeOx, PEtOx and PiPrOx, thereby rendering a PsecBuOx/PMeOx/PEtOx/PiPrOx co-polymer, a combination of PMeOx, PEtOx and PcPrOx, thereby rendering a PsecBuOx/PMeOx/PEtOx/PcPrOx co-polymer, a combination of PMeOx, PEtOx, PnPrOx and PiPrOx, thereby rendering a PsecBuOx/PMeOx/PEtOx/PnPrOx/PiPrOx co-polymer, a combination of PMeOx, PEtOx, PcPrOx and PiPrOx, thereby rendering a PsecBuOx/PMeOx/PEtOx/PcPrOx/PiPrOx co-polymer, a combination of PMeOx, PEtOx, PnPrOx and PcPrOx, thereby rendering a PsecBuOx/PMeOx/PEtOx/PnPrOx/PcPrOx copolymer, a combination of PMeOx, PEtOx, PnPrOx, PiPrOx and PcPrOx, thereby rendering a PsecBuOx/PMeOx/PEtOx/PnPrOx/PiPrOx/PcPrOx co-polymer or any other combination of PMexO, PEtOx, PnPrOx, PcPrOx and/or PiPrOx.

In the following embodiments, different co-polymer compositions are specifically disclosed.

In a specific embodiment, the PAOx of the present invention is a copolymer of 2-ethyl-2-oxazoline and 2-sec-butyl-2-oxazoline (i.e. a PsecBuOx/PEtOx copolymer), such as represented by formula (la)

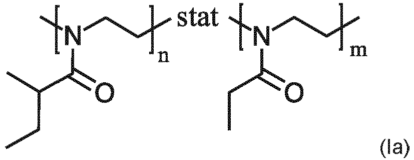

Wherein the sum of m and n is between about 20 - 1000, preferably between 200 - 700, most preferably between 300 - 500.

Formula la thus describes a compound of Formula Z 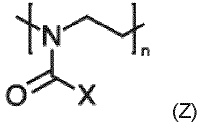 wherein X is ethyl or sec-butyl. The compound of Formula la can also be described as a copolymer of EtOx and secBuOx having different molar ratios of EtOx and secBuOx. This molar ratio can be varied depending on the desired properties of the polyoxazoline.

In a following embodiment, said co-polymer may be represented by formula (Ib) 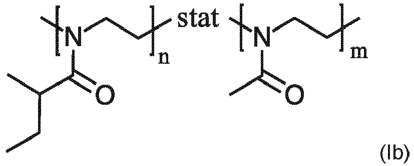 wherein the sum of m and n is between about 20 - 1000, preferably between 200 - 700, most preferably between 300 - 500. Accordingly representing a PsecBuOx/PMeOx copolymer.

In a following embodiment, said co-polymer may be represented by formula (Ic) 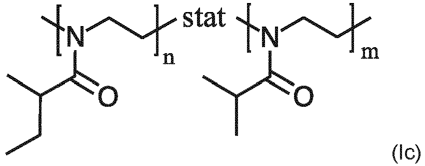 wherein the sum of m and n is between about 20 - 1000, preferably between 200 - 700, most preferably between 300 - 500. Accordingly representing a PsecBuOx/PiPrOx copolymer.

In a following embodiment, said co-polymer may be represented by formula (Id) 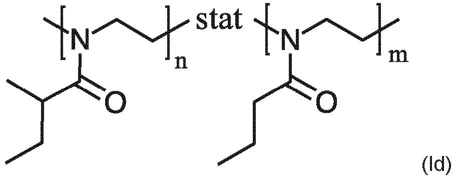 wherein the sum of m and n is between about 20 - 1000, preferably between 200 - 700, most preferably between 300 - 500. Accordingly representing a PsecBuOx/PnPrOx copolymer.

In a following embodiment, said co-polymer may be represented by formula (le) 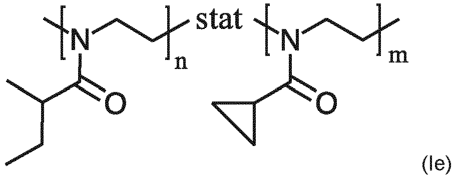 wherein the sum of m and n is between about 20 - 1000, preferably between 200 - 700, most preferably between 300 - 500. Accordingly representing a PsecBuOx/PcPrOx copolymer.

In yet a further embodiment, said co-polymer may be represented by formula (II) 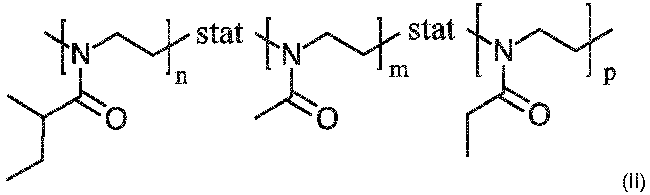 wherein the sum of m, n and p is between about 20 - 1000, preferably between 200 - 700, most preferably between 300 - 500. The combination of secBuOx, EtOx and MeOx in one copolymer structure allows further fine-tuning of the transition temperature of the copolymer, with the hydrophilicity of the monomer increasing in the order secBuOx < nPrOx < cPrOx < iPrOx < EtOx < MeOx, with secBuOx being the least hydrophilic and MeOx being the most hydrophilic. The same principal also applies for any other combination of secBuOx and anyone of EtOx, MeOx, nPrOx, cPrOx and/or iPrOx monomers.

In the following embodiments, different ratio (also termed molar ratio) ranges of monomeric units are disclosed. In the context of the present invention, the ratio of monomeric units can be determined by using the starting materials at the same ratios as desired for the eventual polymers. Accordingly, where a ratio of PsecBuOx monomeric units to PAOx of 5/95 mol% is desired, the polymerisation reaction is performed using 5 mol% of PsecBuOx and 95% of PAOx. After polymerisation, the exact ratio of monomeric units may also be verified using analytical analyses.

In a further embodiment, said co-polymer may comprise a ratio of PsecBuOx monomeric units to PAOx, specifically, PMeOx, PEtOx, PnPrOx, PcPrOx and/or PiPrOx monomeric units of about and between 5/95 mol% and 95/5 mol%; in particular about and between 10/90 mol% and 90/10 mol%, such as about and between 10/90 to 60/40, in particular about 20/80, 30/70 or 50/50. In a more particular embodiment, the ratio is between 20/80 mol% and 80/20 mol%; even more in particular between 30/70 mol% and 70/30 mol%. In the present context, the phrase about and between is also meant to comprise from A to B; in particular about and between 10/90 and 90/10, is meant to be: from 10/90 to 90/10.

In yet another embodiment, said co-polymer may comprise a ratio of PsecBuOx monomeric units to PMeOx, PEtOx, PnPrOx, PcPrOx and/or PiPrOx monomeric units of about and between 50/50 - 80/20 mol%; in particular about and between 55/45 mol% and 75/25 mol%, more in particular between 60/40 mol% and 70/30 mol%; even more in particular between 60/40 mol% and 65/35 mol%.

In a next embodiment, said co-polymer may comprise a ratio of PsecBuOx monomeric units to PMeOx, PEtOx, PnPrOx,PcPrOx and/or PiPrOx monomeric units of about and between 20/80 - 50/50 mol%; in particular about and between 25/75 mol% and 45/55 mol%, more in particular between 30/70 mol% and 40/60 mol%; even more in particular between 35/65 mol% and 40/60 mol%.

The number average molecular weight of the polyoxazoline of the invention is preferably from 10 to 200 kg/mol, more preferably from 30 to 100 kg/mol. The number average molecular weight also termed number average molar mass represents the average of the molecular masses of the individual macromolecules. It is determined by measuring the molecular mass of n polymer molecules, summing the masses and dividing it by n. The number average molecular mass of a polymer can be determined by various techniques such as but not limited to gel permeation chromatography, viscometry, vapor pressure osmometry, end-group determination or proton NMR.

With regard to said monomeric unit ratios, in some embodiments, the co-polymer may become water soluble at temperature below around 20°C, having concentrations of about 30% EtOx. In some embodiments, the co-polymer may become water soluble below around 30°C, having concentrations of about 50% EtOx. In other embodiments, the co-polymer may become water soluble between 40-70°C, having concentrations of at least 50% EtOx.

Alternatively, different amounts of MeOx, EtOx, nPrOx, cPrOx iPrOx or any combination of said monomers may be used to vary the hydrophobic properties of the co-polymers of the present invention, and accordingly their transition temperature, depending on the envisaged applications.

It is a great advantage that varying the ratio of monomeric units allows for a fine control of the transition temperatures. After all, it is desirable in many application domains that such copolymers can be finely controlled in terms of transition temperature given the fact that minor changes in transition temperatures may greatly affect the suitability thereof. Temperatures above said transition temperature result in stable and mechanically robust co-polymer structures, whereas temperatures below said transition temperature results in co-polymers having a high water solubility and, hence, a high hydrophilicity. Depending on the specific application domains, said co-polymers may be tweaked in order to achieve the desired properties at specific temperature ranges. In some embodiments, sharp transitions from a highly hydrophobic to a highly hydrophilic state allow for a rapid dissolution of the co-polymer in the hydrophilic state.

Unless provided otherwise, the term "transition temperature", also referred to as LCST (lower critical solution temperature) should be understood as a temperature at which a material acquires or loses a distinctive property. In this case, it specifically concerns the transition between hydrophobic and hydrophilic properties of a material upon cooling. For example, when a co-polymer reaches or exceeds its transition temperature, the material acquires hydrophobic properties (during heating) or hydrophilic properties (during cooling). For example, when the transition temperature of a co-polymer would be 25°C, the co-polymer may have hydrophobic properties at physiological temperatures (e.g. 37°C) but hydrophilic properties on or below 25°C. Accordingly, where appropriate, the sacrificial template may be removed by placing the template in an aqueous medium (such as water, PBS, cell culture medium) and decreasing the temperature of the template to below the LCST of the polyoxazoline polymer. This temperature is generally below 10 °C, e.g. from 2 to 10 °C

It is an advantage of the current invention that the co-polymer may have hydrophobic properties at physiological temperatures.

Unless provided otherwise, the term "physiological temperatures" (may also be referred to as: normothermia) should be understood as a temperature range generally coinciding with the temperature range found in vertebrates, in particular humans in normal situations. Typically, this range is 36.5-37.5°C. When temperatures deviate from this range, a state of hypothermia (<35°C) or fever (>37.5°C) may be reached.

When applying the concept of controlling said transition temperatures in the current invention, co-polymers may be composed to meet specific needs. For instance, if such co-polymers would be used in the manufacturing of sacrificial templates, benefitting from co-polymers being hydrophilic at specific temperature ranges when e.g. using said sacrificial templates for cell-culture scaffolds.

Unless provided otherwise, the term "sacrificial template" (may also be referred to as: "scaffold") should be understood as a temporary supporting structure which is used in tissue engineering in order to contribute to the formation of new viable/functional tissue for medical purposes. The sacrificial template is regarded as a temporary structure since it can be degraded without surgical removal after it has performed its function. A number of different synthesis methods can be applied in order to prepare sacrificial templates for tissue engineering purposes, such as e.g. electrospinning, melt electrospinning (MES), melt electrowriting, additive manufacturing, fused deposition modelling, thermoforming, casting and 3D printing.

Sacrificial templates may be made in various shapes and forms, such as filaments, fibres, cylinders or films. The template can be created with known methods to deposit polymers such as additive manufacturing (3D printing). These methods include electrospinning, fused deposition modelling, thermoforming and casting. A particular method of creating a microfiber template is melt electrospinning writing (MEW), see for instance Robinson et al., 2019. Typical parameters for this method are a temperature of the polyoxazoline from 190 to 210 °C, flow rate of 0.5 to 0.05 ml/hr, a voltage of +/- 2.5kV to +/- 10 kV, a working distance of 5 mm to 20 mm, and a spinning speed of 8 to 100 mm/s.

A method of creating a filament template is fused deposition modelling (FDM), commonly referred to as 3D printing. This can be used alone to generate filament template structures or combined with MEW to create a multiscale template. Typical parameters for this method are a temperature of the polyoxazoline from 190 to 220 °C, applied extrusion pressure of 1 Bar to 5 Bar, and a deposition speed of 0 to 25 mm/s.

Unless provided otherwise, the term "tissue engineering" should be understood as using a number of methods and materials (e.g. cells) in order to improve or replace biological tissues (e.g. bone, cartilage and blood vessels). Generally, this technique involves the use of a tissue temporary scaffold. The sacrificial templates of the present invention may also be used in the preparation of molds generally, such as for tissue engineering specifically. Upon hardening of the molding material surrounding (parts of) the sacrificial templates, the templates are dissolved and a mold taking the shape of the sacrificial templates remains.

In a further embodiment, a composition comprising said co-polymer is disclosed. In some embodiments, the state of matter of said composition may e.g. be selected from the list comprising: solid, liquid, gas or plasma.

It is beyond dispute that these co-polymers and their unique properties may be of used in compositions also comprising other components. In what follows, a non-limitative number of specific uses are disclosed, benefitting from the specific characteristics of compositions comprising said co-polymer.

In a further embodiment, the present invention provides the compositions and/or copolymers as defined herein for use in human or veterinary medicine.

In yet another embodiment, the present invention provides the compositions and/or copolymers as defined herein for use in manufacturing of a 2D or 3D structure; more in particular in the manufacturing of a sacrificial template. When creating such structures, the specific properties of the building bricks thereof will greatly influence the final structural properties e.g. the stability, mechanical robustness of the final product.

Sacrificial templates are especially useful in the field of tissue engineering and regenerative medicine due to their ability to provide for a structural support for cell attachment and tissue development.

Besides the fact that the materials should accommodate cell attachment and cell differentiation within these structures, it is also of importance that such structures are removable the moment when their scaffolding purpose has been fulfilled (e.g. when the cellular structures are sufficiently dense and strong). When using a composition/co-polymer of embodiments of the current invention in the manufacturing of sacrificial template structures, it is therefore an advantage that (a composition comprising) such a co-polymer may be removable "on-demand". In this case, "on-demand" refers to the possibility of removing the co-polymer by means of changing the properties thereof to achieve a hydrophilic state in order for the copolymer to become rapidly dissolvable (e.g *ex vivo*). More specifically, the ability of said copolymers of rapidly switching between hydrophobic and hydrophilic properties by varying temperature offers great advantages, since e.g. the duration of temperature changes can be reduced to a minimum, which reduces the risk of e.g. cellular damage and allows faster processing. Furthermore, in some embodiments these co-polymers are able to promote cell adhesion and growth in the hydrophobic state without the need of adding any biological material (e.g. collagen).

In a next embodiment, the present invention provides the compositions and/or copolymers as defined herein for use in a method selected from the list comprising: electrospinning (ES), melt electrospinning (MES), melt electrowriting, additive manufacturing and 3D printing. The term solid freeform fabrication (SFF) can sometimes also be used as a collective name for techniques such as MES and MEW when applied e.g. in tissue engineering and scaffold fabrication within the field of biofabrication and bioprinting.

Unless provided otherwise, the term "electrospinning" should be understood as a fiber production method wherein electric force is applied in order to draw charged threads having diameters in the nanometer to micrometer range (e.g. 100 nanometer to several micrometer). When e.g. polymer solutions are used as starting product, it is an advantage of the current method that coagulation chemistry nor high temperatures are needed to produce solid threads therefrom.

Unless provided otherwise, the term "melt electrospinning" should be understood as a fibrous structure production technique wherein polymer melts or polymer solutions are generally used for application such as tissue engineering and filtration.

Unless provided otherwise, the term "melt electrowriting" (MEW) should be understood as the use of straight melt electrospun fibres which are deposited in a layer upon layer approach. Therefore, MEW can be considered a class of 3D printing.

It is of great importance that the printed materials have finely tunable parameters in order to produce stable jets during 3D printing. Therefore, the highly tunable hydrophobic/hydrophilic properties of the co-polymers of the current invention make these co-polymers very suitable for 3D printing applications such as MEW.

In all of the mentioned techniques, (compositions comprising) co-polymers according to embodiments of the invention are thus deployable as 3D printable material (e.g. in 3D bioprinting). In this case, it may serve as a 3D-cell culture grid allowing for cell growth and cell interaction after printed in a specific three-dimensional shape.

All in all, (Compositions comprising) said co-polymers are materials for these solid freeform fabrication (SFF) techniques (e.g. MES, MEW), having the great advantage of determining the monomeric units of these co-polymers in advance in order to achieve an optimized, highly stable, co-polymer which is not associated with any significant loss of material shape nor swelling or hysteresis when applied in said techniques.

It is a further advantage of the current invention that the co-polymer permits melt electrowriting enabling resolutions as low as circa 5 micron.

In a following aspect, use of anyone of the composition and/or co-polymer of the present invention in tissue engineering, implant manufacturing, *in vitro* cell cultures and/or the manufacturing of an in vitro cell-culture scaffold is disclosed.

It is an advantage that the use of (compositions comprising) said co-polymers may be used in three-dimension cell culture systems, since these systems are indispensable for various purposes such as (*in vitro*) disease modelling and drug target identification. The use of e.g. scaffold-based cultures allows for the mimicking of morphological, functional and microenvironmental cellular aspects.

In a next aspect, use of anyone of said composition and/or said co-polymer as a coating is disclosed. These polymer coating materials are thin layers of polymers which can be applied to different types of surfaces. In the present context, the term coating is meant to at least cover absorbed coatings (e.g. physisorption of co-polymers having PsecBuOx-stat-PAOx side chains), spin-coated layers (e.g. spin-coating/doctor) as well as covalently coupled coatings (e.g. covalent coupling of PsecBuOx-stat-PAOx copolymers onto reactive substrates).

In some embodiments, coatings of (compositions comprising) said co-polymers can be applied in order to provide for e.g. functional (hydrophobic water-repellant), protective (e.g. anticorrosive) and/or decorative properties.

Besides that, in some embodiments, coatings of (compositions comprising) said co-polymers can be applied for a variety of biomedical applications. Examples of such biomedical applications include but are not limited to: orthopaedic materials, cardiovascular stents, antibacterial surfaces, tissue engineering and biosensors.

With this respect, said polymer coatings may bestow a wide range of functionalities due to their specific properties, such as e.g. high mechanical strengths and biocompatibility.

In yet another aspect, use of anyone of said composition and/or said co-polymer as a drug formulation is disclosed. Particularly interesting drug formulation comprising the compositions and/or co-polymers of the present invention include but are not limited to solid dispersions, temperature switchable release systems, sustained release formulations...

Depending on the hydrophobic properties of the co-polymers, different types of formulations may be attractive. For example, the more hydrophobic co-polymers may be well suited for the preparation of sustained release formulations, whereas the more hydrophilic co-polymers are interesting for use in solid dispersions having an enhanced solubility. Co-polymers having intermediate hydrophobicity may be well suited for use in temperature switchable release systems.

In a particular embodiment, said drug formulation may be a sustained release formulation. Sustained release formulations are e.g. used for drugs with a small therapeutic window or a short half-life, since safe yet effective therapeutic plasma levels can more easily be achieved. Besides that, multiple daily administrations may be avoided using sustained release formulations. However, formulating such drug products is challenging e.g. in order to be able to guarantee a suitable and constant drug release rate and, hence, to avoid burst releases.

It is an advantage of the current invention that the co-polymer serves as an excellent non-toxic carrier for drug delivery, such as oral drug delivery. Specific advantages include a good stability, a low toxicity and immunogenicity, large loading capacities, and highly tunable hydrophilic and hydrophobic properties. More specifically, the co-polymer is particularly useful in the formulation of oral sustained release formulations comprising one or more active ingredients.

In some embodiments, said composition and/or said co-polymers may be used as a drug carrier for sustained release of one or more active pharmaceutical ingredients.

In some embodiments, the composition and/or said co-polymers and one or more active ingredients may be combined using common formulation methods including but not limited to hot melt extrusion, direct compression, injection moulding, melt granulation or a combination of those, preferably using direct compression or injection moulding.

In some embodiments, said composition comprising said co-polymers may be a polymer mixture in which at least two polymers are blended.

A next aspect relates to a 2D or 3D structure comprising said co-polymer.

As mentioned before, in some embodiments said structures may comprise sacrificial templates.

A next aspect relates to a coating comprising said co-polymer.

In some embodiments, different co-polymers having different ratios of monomeric units may be combined in a single coating.

### EXAMPLES

### EXAMPLE1:

To evaluate polymers suitable in the preparation of a sacrificial template, the polymers were submitted to thermally triggered dissolution. To perform this test, large filaments were extruded (approximately 1 mm in diameter) using the FDM (fused deposition modeling) method described above with a 150 µm diameter nozzle, a temperature of 200°C and 5 Bar of applied pressure. The system used to extrude was a Bioinicia LE-100 Electrospinning system with custom MEW hardware consisting of a band heater controlled with a Temptron PID controller, which heats a metal syringe that is supported above the flat collector from the XY gantry system. These were placed within a Peltier heating/cooling element capable of maintaining liquid at temperatures ranging from 50 °C to approximately 4 °C. This test allows to emulate the intended process flow where cells are seeded on the template at 37 °C. And then the entire device is placed in a standard refrigerator (typically at 5 °C) to trigger template dissolution.

### Comparative example PnPrOx

Poly(2-n-propyl-2-oxazoline) with a molecular weight of 50 kg/mol was tested. This polymer has a LCST of about 30 °C. Solubility was tested in a PBS solution. A filament was prepared via FDM as described above. Briefly, the polymer was heated to 200°C within a metal syringe and extruded through a 150 µm diameter brass 3D printing nozzle with 5 Bar of air pressure. The filament was exposed to 37 °C for 10 minutes and then rapidly cooled to 5 °C.

While the filament is maintained at 37°C, one can observe a change in filament opacity as it slowly absorbs some water but still maintains mechanical and morphological integrity. During the cooling process, one can observe the filament becoming rapidly more translucent as the material becomes increasingly more hydrophilic and, therefore, more soluble. However, it was observed that complete dissolution was only achieved by maintaining the filament at 5°C for approximately 3 hrs.

In order to emulate a cell seeding process, whereby the template scaffold is seeded with cells prior to embedding and dissolution, we studied if the filament could be maintained at 37°C for an extend period.

After maintaining the filament for 1hr at 37°C, it was found that the filament no longer dissolved. This was ascribed to be a consequence of structural reorganization, i.e. of the semicrystalline character of this polymer, resulting in partial crystallization. By maintaining the polymer at 37°C (close to its Tg = 40°C), the side changes were able to reorganize the fiber surface leading to hydrophobic fibres that can no longer dissolve.

A follow up experiment used smaller MEW generated fibres (approximately 20 µm) using a 150 µm diameter 3D printing nozzle, a temperature of 190°C, 0.25 Bar of pressure, -4 kV of applied voltage, 5 mm working distance, and a translation speed of 75 mm/s. Observing these fibres under similar dissolution conditions found that this phenomenon was consistent and not dependent on fibre/filament size or differences in surface-to-volume ratio (data not shown). For both sizes, samples were kept in the refrigerator for 3 days and the material still did not dissolve (data not shown).

### Further comparative examples

Further polyoxazoline variants were tested. The polymers had a number average molar mass above 30 kg/mol and were processed into fibers, using a 150 µm diameter 3D printing nozzle as described above for PnPrOx. The thermoresponsive dissolution behavior of the polymers was investigated in water with the following outcomes:
PcPrOx: Poly(2-c-Propyl-2-oxazoline); LCST ~30 °C
Fast dissolution upon contact with water at 37 °C, not suitable
PEtOx-stat-PnPrOx: Poly(2-ethyl-2-oxazoline)-stat-Poly(2-n-propyl-2-oxazoline; LCST 24-60 °C
Fast dissolution upon contact with water when above the LCST, not suitable.
PEtOx-stat-PnBuOx: Poly(2-ethyl-2-oxazoline)-stat-Poly(2-n-butyl-2-oxazoline); LCST 20-30°C:
   Fast dissolution upon contact with water above the LCST at 37°C or 42°C , not suitable.
   PsecBuOx: Poly(2-sec-Butyl-2-oxazoline) LCST ~5 °C
   No dissolution in water at 5 °C, not suitable.

Further to these comparative examples, a copolymer of PEtOx and PsecBuOx in accordance with the present invention was made with a molar ratio of 30/70 and an LCST of ~20°C - Poly(2-ethyl-2-oxazoline)-stat-poly(2-sec-butyl-2-oxazoline) (PEtOx-stat-PsecBuOx 30/70). A filament was made of this copolymer as described above using a 150 µm diameter brass 3D printing nozzle connected to a metal syringe heated to 205°C through which the molten polymer was extruded with 5 Bar of air pressure. Immersed in 37°C PBS, the filament of this polymer maintained both its shape and mechanical properties. It also retained air bubbles on its surface, indicating hydrophobicity. This was maintained for 10 minutes, after which a rapid cooling phase showed the polymer beginning to change shape, losing the air bubbles on the surface, and then began to dissolve starting at around 20°C, close to the polymer TCP. When the polymer is maintained at 5°C, dissolution is complete after 5 minutes.

This dissolution assay shows that this polymer has the desired properties. A test of PEtOx-stat-PsecBuOx 30/70 kept at 37°C overnight in PBS showed that the polymer still dissolved at 5°C.

### EXAMPLE 2:

The monomer purification and polymer synthesis methodologies in the below examples were performed as reported elsewhere.

### Monomer synthesis and purification

2-Ethyl-2-oxazoline (EtOx; Polymer Chemistry Innovations) was purified *via* fractional distillation and purification over barium oxide. 2-sec-butyl-2-oxazoline (secBuOx) was synthesized *via* the Witte-Seeliger method (Witte et al., Ann. Chem. 1974), from their corresponding nitrile, i.e. 2-methylbutyronitrile. The purification of secBuOx was carried out similarly to that of EtOx.

### Initiator

Trifluoromethanesulfonic acid was purchased from Sigma Aldrich and used as received.

### Polymerization

Polymers were synthesized with a target number of repeating units typically from 300 to 500. A typical polymer synthesis involves the administration of secBuOx monomer and a comonomer, such as EtOx, in a microwave reaction vial under an inert atmosphere. Both monomers are dosed in the desired molar ratio. Subsequently, the initiator is added in the required quantity to match the desired polymer length.

The vial is sealed under an inert atmosphere and placed in a microwave reactor (Biotage Initiator) at a temperature of 120 °C for 60 minutes.

Representative example of a polymerization: Poly[(2-ethyl-2-oxazoline)₁₂₀-stat-(2-sec-butyl-2-oxazoline)₂₈₀ (PEtOx₁₂₀-stat-PsecBuOx₂₈₀)

An oven dried 20 mL microwave reactor vial is transferred to a glovebox (Vigor technologies) with a water content below 0.1 ppm. The vial is loaded with a stirring bar, 3.060 mL of EtOx (3.005 g, 30.3 mmol) and 9.67 mL of sec-BuOx (8.99 g, 70.7 mmol). The vial is closed and transferred out of the glovebox. A 25 mL Schlenk flask is dried, fitted with a septum, connected to a Schlenk line and filled with Argon. 10 mL of dry acetonitrile are injected into the flask, followed by 0.800 mL of trifluoromethanesulfonic acid. This stock solution is homogenized, and 0.279 mL initiator (0.038 g., 0.25 mmol) are taken with a syringe. The solution is then injected into the microwave vial containing the monomer mixture. The vial is placed in the microwave synthesizer and heated to 120 °C for 60 minutes.

### Purification and characterization

The synthesized polymers were dissolved in dichloromethane and purified by washing three times with a saturated solution of NaHCO₃ and once with water.

The polymers where characterized by 1H-NMR spectroscopy and size exclusion chromatography (SEC) on an Agilent 1260-series HPLC system equipped with a 1260 online degasser, a 1260 ISO-pump, a 1260 automatic liquid sampler (ALS), a thermo-stated column compartment (TCC) at 50 °C equipped with two PLgel 5 µm mixed-D columns in series, a 1260 diode array detector (DAD) and a 1260 refractive index detector (RID). The used eluent is N,N-dimethylacetamide (DMA) containing 50 mM of lithium chloride at an optimized flow rate of 0.5 mL/min. The spectra were analyzed using the Agilent ChemStation software with the GPC add on. Molar mass (Mn and Mp) and dispersity ( 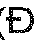 ) values were calculated against polymethylmethacrylate molar mass standards from PSS.

The characterization data for the synthesized polymers is summarized in Table 2.

**Table 2. Overview size-exclusion chromatography data for the PAOx copolymers.**

| Batch | Mₚ (PMMA) | Mₙ (PMMA) | Ð | TCP |
|---|---|---|---|---|
| | kDa | kDa | - | °C |
| PEtOx₈₀-stat-PsecBuOx₃₂₀ | 49,200 | 28,100 | 2.04 | 13 |
| PEtOx₁₂₀-stat-PsecBuOx₂₈₀ | 53,000 | 38,600 | 1.97 | 22 |
| PEtOx₂₀₀-stat-PsecBuOx₂₀₀ | 101,900 | 43,200 | 2.16 | 30 |
| PsecButOx₃₀₀ | 54,000 | 63,000 | 1.14 | < 4 |

### Evaluation of polymer thermoresponsive properties

The synthesized polymers were suspended in distilled water targeting a polymer concentration of 5.0 mg/mL. The suspensions were immersed in an ice bath and shaken regularly until complete polymer dissolution was observed. All the synthesized PsecBuOx copolymers, including PsecBuOx homopolymers, were soluble in ice water. However, the Tcp of the homopolymer was found to be below 4 °C.

The determination of the cloud point temperature of the samples was performed in a Crystal 16 turbidimeter (Avantium Technologies). 1.6 mL of each polymer solution was taken into a 2.0 mL vial. The vials were heated in a ramp from 5 to 60 °C at a rate of 1 K/min. The observed cloud point temperatures are reported in Table 2, and the corresponding curves shown in Figure 1. This figure relates to temperature-dependent turbidimetry measurements - 2^{nd} heating ramp- of PEtOx₂₀₀-stat-PsecBuOx₂₀₀ (a) showing a Tcp of ca. 30 °C, PEtOx₁₂₀-stat-PsecBuOx₂₈₀ (b) showing a Tcp of ca. 20 °C, and PEtOx₈₀-stat-PsecBuOx₃₂₀ (c) showing a Tcp of ca. 13 °C according to an embodiment of the current invention. The % transmission in (b) fluctuates due to the formation of macroscopic aggregates that deposit in the bottom of the vial.

Water contact angle (WCA) was measured to determine the hydrophobicity of the polymer at different temperatures. This confirms the LCST behavior and also allows one to estimate the ability of cells to adhere to the polymer surface, since it is widely recognized that cells prefer a moderately hydrophobic surface (40° to 60° WCA). This was measured by spin coating a thin film of the polymer (dissolved in chloroform) onto a glass coverslip and then placing this coating coverslip onto a flat Peltier element. This was incorporated into a WCA measurement system along with a heated syringe of PBS which maintained PBS solution at approximately 60°C. A drop of this warmed PBS was deposited onto the polymer film and the WCA was monitored via time lapse over a 2 minute period until the angle had stabilize. The angle was automatically according to the proprietary software of the WCA system. The WCA of the last 30 seconds was observed to be stable and, therefore, averaged to produce the 'final' WCA. This was measured for a number of temperatures ranging from 37°C to 5°C, reflecting the transition from cell incubator to refrigerator, respectively.

Data for the copolymer PEtOx₁₂₀-stat-PsecBuOx₂₈₀ were compared with state of the art polymer poly(N-isopropylacrylamide) (PNIPAM) and a different poly(2-oxazoline) not according to the invention: poly(2-n-propyl-2-oxazoline, (PnPrOx) with a Mw of 50 kDa. The results are shown in Figure 2.

Further experiments were done to evaluate cell seeding efficiency. After preparing a solution of PEtOx₁₂₀-stat-PsecBuOx₂₈₀ in water, this was added to a tissue culture well plate and the water was allowed to evaporate, forming a thin film on the bottom of each well. Primary rat Schwann cells were seeded in each well and allowed to adhere and grow over a 3-day period. Cells appeared to adhere well, though the cell morphology was not comparable to normal tissue culture plastic.

After seeding and maintenance for 3 days, the well plate was cooled to 4 °C for 15 minutes to allow the polymer to dissolve. The culture medium was collected and spun down to collect the cells in the bottom of a 15 ml tube. Cells were carefully collected, resuspended in clean medium and replaced in a fresh well plate. They were observed to adhere again, indicating that they had survived the process and remained viable.

### Fiber formation

The polyoxazoline of the invention is used to create a template. The shape of the template is not particularly restricted and can be varied depending on the particular structure for cell growth that is created.

The template can be created with known methods to deposit polymers. These methods include electrospinning, fused deposition modeling, thermoforming and casting.

A particular method of creating the template is melt electrospinning writing (MEW).

Typical parameters for this method are a temperature of the polyoxazoline from 190 to 210 °C and a spinning speed of 8 to 100 mm/s.

Fibres were manufactured with MEW with diameters from 15 to 20 µm. With a 150 µm diameter brass 3D printing nozzle, a deposition speed of 50 mm/s, a voltage of -7kV, working distance of 10 mm, and a pressure of 1 Bar (100 kPa), temperatures were varied resulting in the following fibre diameters.

| **Temp (°C)** | **Diameter (µm)** |
|---|---|
| 193 | 14 ± 0.14 |
| 196 | 15 ± 0.1 |
| 200 | 18 ± 0.05 |

Further dissolution data were obtained for the polymers shown below in Table 3

**Table 3**

| | Polymer PEtOx/PsecBuOx | Polymer Format | Preincubation Temp. | Preincubation Time | Swelling Temp. | Dissolution Temp. | Dissolution Time |
|---|---|---|---|---|---|---|---|
| 1 | 30:70 | Filament | 37°C | 10 min | 19°C | 5°C | 18 min |
| 2 | 30:70 | Fibers | 37°C | 10 min | 29°C | 18°C | 9 min |
| 3* | 30:70 | Fibers | 37°C | 16 hrs | n/a | 4°C | 30 min |
| 4* | 20:80 | Fibers | 21°C | 10 min | n/a | 4°C | ~1 hr |

| | | | | | | | |
|---|---|---|---|---|---|---|---|
| *These experiments were performed in an application setting, where the incubations and temperatures were applied as it would be during template use. | | | | | | | |

The viscosity of the polymer melt determines the flow rate for a pressure driven MEW system, were flows from 0.05 to 0.5 mL/h are achieved by applying pressures from 0.5 to 1.5 Bar. For a more defined process parameter, melt viscosity was measured for the different PEtOx-stat-PsecBuOx variants with a parallel plate rheometer with heated plates. 100 mg of polymer were loaded between the plates, the temperature was raised to 200°C to initially melt the polymer, and then the viscosity was determined by measuring the rotational force required to subject the polymer melt to a cyclic of 1° angular displacement. The sample was then cooled slowly by 1°C/min. This data shows that, for the same typical range of operating temperatures (from 190 to 200°C) the PEtOx₁₂₀-stat-PsecBuOx₂₈₀ and PEtOx₈₀-stat-PsecBuOx₃₂₀ achieve approximately similar melt viscosity. For the PEtOx₂₀₀-stat-PsecBuOx₂₀₀, a much higher viscosity is measured for the same range, indicating that lower flow rates will be generated and that higher temperatures (~225°C) are required for this polymer to be processed in a similar manner. The results are shown in Figure 3.

### Evaluation of polymer fiber behavior in an aqueous environment

The produced fibers were immersed in a thermostated bath. At temperatures above the polymer phase transition temperature, the fibers maintained their morphology and structural integrity.

### REFERENCES

Halperin, A., et al. Poly (N-isopropylacrylamide) Phase Diagrams: Fifty Years of Research. Angewandte Chemie International Edition 2015, 54(51): 15342-15367.

Oleszko-Torbus, N., et al. W. Poly(2-oxazoline) Matrices with Temperature-Dependent Solubility-Interactions with Water and Use for Cell Culture. Materials 2020, 13, 2702.

Robinson, TM., et al. The Next Frontier in Melt Electrospinning : Taming the Jet. Adv Funct Mater. 2019;1904664.

Ryma, M., et al. Easy-to-Prepare Coating of Standard Cell Culture Dishes for Cell-Sheet Engineering Using Aqueous Solutions of Poly(2-n-propyl-oxazoline). ACS Biomater. Sci. Eng. 2019, 5, 3, 1509-1517.

Schoolaerts E., et al. Waterborne Electrospinning of Poly(N-isopropylacrylamide) by Control of Environmental Parameters. ACS Appl. Mater. Interfaces 2017, 9, 28, 24100-24110.

Van Durme, K., et al. Kinetics of Demixing and Remixing in poly(N-isopropylacrylamide)/Water Studied by Modulated Temperature DSC. Macromolecules 2004, 37, , 25, 9596-9605.

## Claims

1. A PsecBuOx-stat-PAOx co-polymer represented by formula (I); 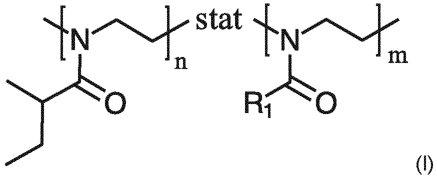 wherein
each R₁ is independently selected from -methyl, -ethyl, -n-propyl, cyclo-propyl and iso-propyl; stat denotes a statistical copolymer, meaning a co-polymer formed by one-pot statistical copolymerization of the monomer units disclosed in formula (I);
the sum of m and n is between 20 - 1000, preferably between 200 - 700, most preferably between 300 - 500.

2. The co-polymer as defined in claim 1 and represented by formula (la) 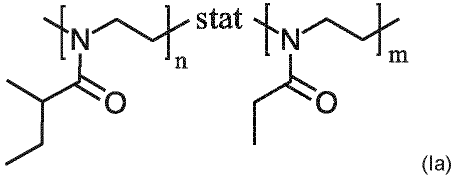 wherein
the sum of m and n is between 20 - 1000, preferably between 200 - 700, most preferably between 300 - 500.

3. The co-polymer as defined in claim 1 and represented by formula (Ib) 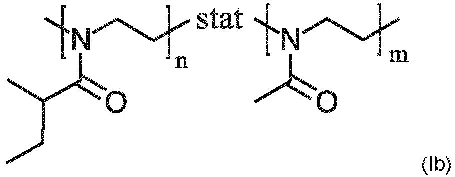 wherein
the sum of m and n is between 20 - 1000, preferably between 200 - 700, most preferably between 300 - 500.

4. The copolymer according to anyone of claims 1-3, wherein the ratio of PsecBuOx monomeric units to PMeOx, PEtOx, PnPrOx, PcPrOx and/or PiPrOx monomeric units is about and between 5/95 mol% and 95/5 mol%.

5. The copolymer according to anyone of claims 1-3, wherein the ratio of PsecBuOx monomeric units to PMeOx, PEtOx, PnPrOx, PcPrOx and/or PiPrOx monomeric units is about and between 50/50 - 80/20 mol%.

6. The copolymer according to anyone of claims 1-3, wherein the ratio of PsecBuOx monomeric units to PMeOx, PEtOx, PnPrOx, PcPrOx and/or PiPrOx monomeric units is about and between 20/80 - 50/50 mol%.

7. A composition comprising a copolymer as defined in anyone of claims 1 to 6.

8. The copolymer as defined in anyone of claims 1 to 6; or the composition as defined in claim 7 for use in human or veterinary medicine.

9. Use of the copolymer as defined in anyone of claims 1 to 6; or the composition as defined in claim 7 in the manufacturing of a 2D or 3D structure; more in particular in the manufacturing of a sacrificial template.

10. Use of the copolymer as defined in anyone of claims 1 to 6; or the composition as defined in claim 7 in a method selected from the list comprising: electrospinning (ES), melt electrospinning (MES), melt electrowriting, additive manufacturing, fused deposition modelling, thermoforming, casting and 3D printing.

11. Use of the copolymer as defined in anyone of claims 1 to 6; or the composition as defined in claim 7 in tissue engineering, implant manufacturing, *in vitro* cell cultures, and/or the manufacturing of an in vitro cell-culture scaffold.

12. Use of the copolymer as defined in anyone of claims 1 to 6; or the composition as defined in claim 7 as a coating.

13. Use of the copolymer as defined in anyone of claims 1 to 6; or the composition as defined in claim 7 as a drug formulation.

14. A 2D or 3D structure comprising the co-polymer as defined in anyone of claims 1 to 6.

15. A coating comprising the co-polymer as defined in anyone of claims 1 to 6.

## Patentansprüche

1. PsecBuOx-stat-PAOx-Copolymer, dargestellt durch Formel (I); 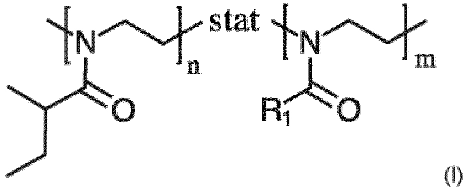 wobei
jedes R₁ unabhängig ausgewählt ist aus -Methyl, -Ethyl, -n-Propyl, Cyclopropyl und Isopropyl;
stat ein statistisches Copolymer, das heißt ein Copolymer bezeichnet, das durch statistische Ein-Topf-Copolymerisation der in Formel (I) offenbarten Monomereinheiten ausgebildet wird;
die Summe von m und n zwischen 20-1000, vorzugsweise zwischen 200-700, am meisten bevorzugt zwischen 300-500 liegt.

2. Copolymer wie in Anspruch 1 definiert und dargestellt durch Formel (la), 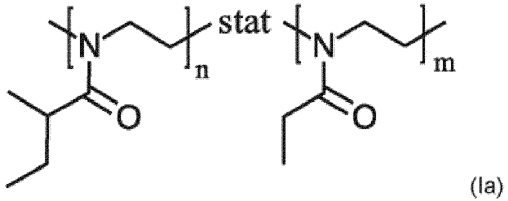 wobei
die Summe von m und n zwischen 20-1000, vorzugsweise zwischen 200-700, am meisten bevorzugt zwischen 300-500 liegt.

3. Copolymer wie in Anspruch 1 definiert und dargestellt durch Formel (Ib), 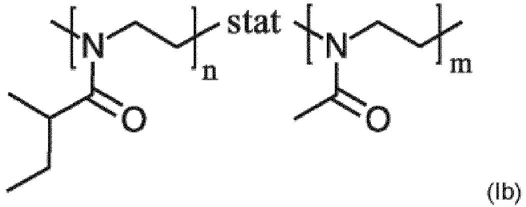 wobei
die Summe von m und n zwischen 20-1000, vorzugsweise zwischen 200-700, am meisten bevorzugt zwischen 300-500 liegt.

4. Copolymer nach einem der Ansprüche 1 bis 3, wobei das Verhältnis von PsecBuOx-Monomereinheiten zu PMeOx-, PEtOx-, PnPrOx-, PcPrOx- und/oder PiPrOx-Monomereinheiten etwa und zwischen 5/95 mol % und 95/5 mol % beträgt.

5. Copolymer nach einem der Ansprüche 1 bis 3, wobei das Verhältnis von PsecBuOx-Monomereinheiten zu PMeOx-, PEtOx-, PnPrOx-, PcPrOx- und/oder PiPrOx-Monomereinheiten etwa und zwischen 50/50-80/20 mol % beträgt.

6. Copolymer nach einem der Ansprüche 1 bis 3, wobei das Verhältnis von PsecBuOx-Monomereinheiten zu PMeOx-, PEtOx-, PnPrOx-, PcPrOx- und/oder PiPrOx-Monomereinheiten etwa und zwischen 20/80-50/50 mol % beträgt.

7. Zusammensetzung, umfassend ein Copolymer wie in einem der Ansprüche 1 bis 6 definiert.

8. Copolymer wie in einem der Ansprüche 1 bis 6 definiert; oder die Zusammensetzung wie in Anspruch 7 definiert, zur Verwendung in der Human- oder Veterinärmedizin.

9. Verwendung des Copolymers wie in einem der Ansprüche 1 bis 6 definiert; oder der Zusammensetzung wie in Anspruch 7 definiert, bei der Fertigung einer 2D- oder 3D-Struktur; weiter insbesondere bei der Fertigung einer Opferschablone.

10. Verwendung des Copolymers wie in einem der Ansprüche 1 bis 6 definiert; oder der Zusammensetzung, wie in Anspruch 7 definiert, in einem Verfahren, ausgewählt aus der Liste, umfassend: Elektrospinnen (ES), Schmelzelektrospinnen (MES), Schmelzelektroschreiben, additive Fertigung, Schmelzschichtmodellierung, Thermoformen, Gießen und 3D-Druck.

11. Verwendung des Copolymers wie in einem der Ansprüche 1 bis 6 definiert; oder der Zusammensetzung wie in Anspruch 7 definiert, in der Gewebezüchtung, der Implantatfertigung, *in-vitro*-Zellkulturen und/oder der Fertigung eines in-vitro-Zellkulturgerüsts.

12. Verwendung des Copolymers wie in einem der Ansprüche 1 bis 6 definiert; oder der Zusammensetzung, wie in Anspruch 7 definiert, als eine Beschichtung.

13. Verwendung des Copolymers wie in einem der Ansprüche 1 bis 6 definiert; oder der Zusammensetzung, wie in Anspruch 7 definiert, als eine Arzneimittelformulierung.

14. 2D- oder 3D-Struktur, umfassend das Copolymer wie in einem der Ansprüche 1 bis 6 definiert.

15. Beschichtung, umfassend das Copolymer wie in einem der Ansprüche 1 bis 6 definiert.

## Revendications

1. Copolymère PsecBuOx-stat-PAOx représenté par la formule (I) ; 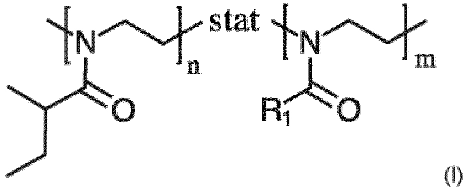 dans lequel
chaque R₁ est indépendamment choisi parmi -méthyle, -éthyle, -n-propyle, cyclo-propyle et iso-propyle ;
stat désigne un copolymère statistique, c'est-à-dire un copolymère formé par copolymérisation statistique monotope des motifs monomères divulgués dans la formule (I) ;
la somme de m et n est comprise entre 20 et 1000, de préférence entre 200 et 700, le plus préférablement entre 300 et 500.

2. Copolymère selon la revendication 1 et représenté par la formule (la) 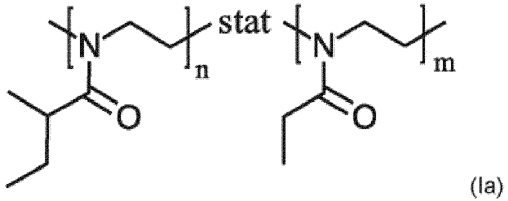 dans lequel
la somme de m et n est comprise entre 20 et 1000, de préférence entre 200 et 700, le plus préférablement entre 300 et 500.

3. Copolymère selon la revendication 1 et représenté par la formule (lb) 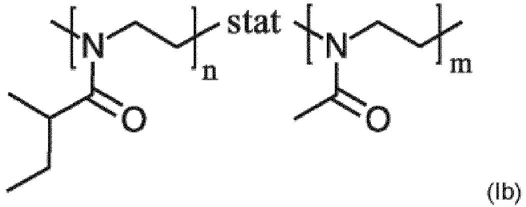 dans lequel
la somme de m et n est comprise entre 20 et 1000, de préférence entre 200 et 700, le plus préférablement entre 300 et 500.

4. Copolymère selon l'une quelconque des revendications 1 à 3, dans lequel le rapport des motifs monomères PsecBuOx aux motifs monomères PMeOx, PEtOx, PnPrOx, PcPrOx et/ou PiPrOx est d'environ et compris entre 5/95 % en moles et 95/5 % en moles.

5. Copolymère selon l'une quelconque des revendications 1 à 3, dans lequel le rapport des motifs monomères PsecBuOx aux motifs monomères PMeOx, PEtOx, PnPrOx, PcPrOx et/ou PiPrOx est d'environ et compris entre 50/50 et 80/20 % en moles.

6. Copolymère selon l'une quelconque des revendications 1 à 3, dans lequel le rapport des motifs monomères de PsecBuOx aux motifs monomères de PMeOx, PEtOx, PnPrOx, PcPrOx et/ou PiPrOx est d'environ et compris entre 20/80 et 50/50 % en moles.

7. Composition comprenant un copolymère selon l'une quelconque des revendications 1 à 6.

8. Copolymère selon l'une quelconque des revendications 1 à 6 ; ou composition selon la revendication 7 pour utilisation en médecine humaine ou vétérinaire.

9. Utilisation du copolymère selon l'une quelconque des revendications 1 à 6 ; ou composition selon la revendication 7 dans la fabrication d'une structure 2D ou 3D ; plus particulièrement dans la fabrication d'un modèle sacrificiel.

10. Utilisation du copolymère selon l'une quelconque des revendications 1 à 6 ; ou composition selon la revendication 7 dans un procédé choisi dans la liste comprenant : l'électrofilage (ES), l'électrofilage à l'état fondu (MES), l'électro-écriture à l'état fondu, la fabrication additive, la modélisation par dépôt de fil fondu, le thermoformage, la coulée et l'impression 3D.

11. Utilisation du copolymère selon l'une quelconque des revendications 1 à 6 ; ou composition selon la revendication 7 dans l'ingénierie tissulaire, la fabrication d'implants, les cultures de cellules *in vitro*, et/ou la fabrication d'un échafaudage de culture de cellules in vitro.

12. Utilisation du copolymère selon l'une quelconque des revendications 1 à 6 ; ou composition selon la revendication 7 en tant que revêtement.

13. Utilisation du copolymère selon l'une quelconque des revendications 1 à 6 ; ou composition selon la revendication 7 en tant que formulation médicamenteuse.

14. Structure 2D ou 3D comprenant le copolymère selon l'une quelconque des revendications 1 à 6.

15. Revêtement comprenant le copolymère selon l'une quelconque des revendications 1 à 6.
